# EUROPÄISCHE PATENTSCHRIFT

(11) **EP 1 322 379 B1**
(45) Veröffentlichungstag und Bekanntmachung des Hinweises auf die Patenterteilung: **19.10.2005**
(21) Anmeldenummer: 01964881.5
(22) Anmeldetag: 10.08.2001
(51) Int. Cl.: A61N 1/32

(54) **ELEKTROTHERAPEUTISCHES GERÄT**
ELECTRO-THERAPEUTIC DEVICE
APPAREIL ELECTROTHERAPEUTIQUE

(30) Priorität: 11.08.2000 DE 10039240
(43) Veröffentlichungstag der Anmeldung: 02.07.2003
(73) Patentinhaber: May, Hans-Ulrich, 75015 Bretten (DE)
(72) Erfinder: May, Hans-Ulrich, 75015 Bretten (DE)
(74) Vertreter: Geitz, Holger
(86) Internationale Anmeldenummer: PCT/DE2001/002983
(87) Internationale Veröffentlichungsnummer: WO 2002/013904

(56) Entgegenhaltungen:
- US-A- 4 243 043
- US-A- 4 922 908
- US-A- 5 350 414
- US-A- 5 573 552
- US-A- 5 718 662

## Beschreibung

Die Erfindung betrifft ein elektrotherapeutisches Gerät zur Behandlung des vorzugsweise menschlichen Körpers mit elektrischen Strömen definierter Frequenz und Amplitude mit wenigstens zwei mit dem zu behandelnden Körper zum Schluß eines Stromkreises über diesen Körper verbindbaren Flächenelektroden, bei dem ein Behandlungsstrom, dessen Amplitude (A) und Frequenz (f) simultan modulierbar ist, in den zu behandelnden Körper einspeisbar ist.

Ein derartiges Gerät ist aus der japanischen Patentanmeldung JP 5-212 126 A vorbekannt.

Ein ähnliches Gerät, wenn auch ohne simultane Modulation von Amplitude und Frequenz ist aus der europäischen Patentschrift EP 0 659 099 B1 vorbekannt.

Bei diesem vorbekannten Elektrotherapiegerät wird zwischen zwei Reizstrommethoden unterschieden:
- das polaritätsabhängige "Polaritäre Reizprinzip "
- das polaritätsunabhängige "Apolaritäre Reizprinzip"

Das polaritäre Reizprinzip gilt für niederfrequente Ströme im Bereich von 0 bis 200 Hz das apolaritäre Reizprinzip gilt für sogenannte mittelfrequente Ströme im Bereich von etwa 1 - 100 kHz.

Bei der Elektrotherapie kann man ferner zwischen frequenzabhängigen und amplitudenabhängigen Reizwirkungen unterscheiden. Es sind ferner Methoden bekannt, die diese Wirkungen isoliert hervorheben bzw. in sinnvoller Weise miteinander kombinieren.

Dabei wird bei den frequenzabhängigen Methoden die Frequenz des Behandlungsstromes und bei den amplitudenabhängigen Methoden die Amplitude des Behandlungsstromes verändert.

Stellt man in einem doppelt-logarithmischen Koordinatensystem die Schwellen als Funktion der Frequenz dar, wobei die Spannungs- bzw. Stromintensität als Ordinaten- und die Frequenzen als Abszissenwerte dargestellt sind, so resultiert ein annähernd geradliniger Kurvenverlauf. Der Kurvenverlauf ist allerdings nur annähernd geradlinig ansteigend, weil mit zunehmenden Frequenzwerten die Intensitätsschwellenwerte relativ stärker zunehmen, so daß die Steigung der Kurve mit zunehmender Frequenz entsprechend ein wenig anwächst. Anders ausgedrückt, die annähernd geradlinig ansteigende Kurve "hängt ein wenig durch".

Der erwähnte nur annähernd geradlinige Kurvenverlauf repräsentiert die Reizschwelle, also einen frequenzabhängigen Intensitätsschwellwert, dessen Überschreiten Aktionspotentiale auslöst.

Die Frequenz, mit der diese Schwelle durch wiederholte Änderung der Amplitude wiederholt überschritten wird, bestimmt die Häufigkeit der Auslösung der genannten Aktionspotentiale bei den amplitudenabhängigen Stimulationsverfahren.

In dem genannten Koordinatensystem bewegen sich die im Bereich der frequenzabhängigen Elektrotherapie eingesetzten Behandlungsströme auf einer horizontalen Geraden und die im Falle der amplitudenabhängigen Behandlung erzeugten Behandlungsströme auf einer vertikalen Geraden.

Die zwei entsprechenden Verfahren werden danach auch als horizontale und als vertikale Stimulation bezeichnet.

In beiden Verfahren sind auch rein unterschwellige Anwendungen bekannt, wenn bei der Therapie auf andere Wirkungen als auf die beschriebenen Stimulationswirkungen Wert gelegt wird.

Die Erfolge der Elektrotherapie betreffen vor allem den Bereich der Schmerzlinderung, der Reizung von quergestreifter und glatter Muskulatur, der Durchblutungsbeeinflussung, der Stoffwechselbeeinflussung, der Entzündungshemmung und Regenerationsförderung im Bereich der Wund- und Knochenheilung.

Die Grundlage für die therapeutische Breite von Strömen sind deren physikalische Parameter und die sich aus diesen ableitbaren Beeinflussungen der Funktionen der Strukturen des behandelten biologischen Systems.

Derartige Stromparameter sind vor allem die Frequenz mit ihren Modulationen und Modulationsfrequenzen und Intensitätsparameter wie Spannung, Stromstärke, Leistung, lokal wirksame Feldstärke, Stromdichte, Leistungsdichte etc. sowie wiederum deren Modulation als Amplitudenmodulation mit Amplitudenmodulationsfrequenz und Modulationsgrad.

Bei Strömen muß wie bei Pharmaka zwischen systemischer und lokaler Verträglichkeit unterscheiden werden.

Die systemische Verträglichkeit von Strömen wird vor allem durch die für die Auslösung von Herzkammerflimmern oder die für die Auslösung von epileptischen Anfällen erforderliche elektrotoxische Dosis bestimmt.

Die "schlechteste" lokale Verträglichkeit zeigt Gleichstrom wegen lokaler Verbrennungs- und Verätzungsgefahr. Niederfrequente Ströme sind wegen ihrer schlechten transkutanen Einkoppelbarkeit und der sich daraus ableitenden niedrigen lokalen Schmerzschwellen auch recht unverträglich. Niederfrequente Ströme haben zudem meist noch eine Gleichstromkomponente mit den erwähnten Problemen.

Die Nachteile niederfrequenter Ströme und von Gleichstrom können bei einer sogenannten "horizontalen Stimulation" im Mittelfrequenzbereich zwischen 1 kHz und 100 kHz gemäß der aus der EP 0 659 099 B1 vorbekannten Vorrichtung vermieden werden. Das Prinzip besteht darin, daß in dem genannten Koordinatensystem eine horizontale niederfrequente Änderung der Trägerfrequenz derart erfolgt, daß zwischen einem über- und einem unterschwelligen Trägerfrequenzbereich variiert wird, d.h., die mittelfrequenten Trägerfrequenzen werden niederfrequent frequenzmoduliert.

Dabei ist auch das Verfahren der horizontalen Stimulation nicht ohne Nachteile. Um die Schwelle einer Gruppe sensibler, motorischer oder sympathischer Nervenfasern zu erreichen, werden die Intensitäten zwischen einer oberen und unteren Eckfrequenz in gleicher Weise erhöht. Mit der unteren Eckfrequenz wird dabei die Schwelle einer Nervenfaser zuerst überschritten. Beim Erreichen der Schwelle ist die Dauer der Überschwelligkeit im Bereich der unteren Trägerfrequenzen noch kurz im Vergleich der Unterschwelligkeit im Bereich der höheren Trägerfrequenzen.

Soll die Anzahl der überschwellig gereizten Nervenfasern erhöht werden, um z.B. über mehr stimulierte motorische Nervenfasern die Intensität der Muskelkontraktion zu erhöhen oder über eine größere Anzahl sensibler Nervenfasern die Intensität der Gegenirritation zur Schmerzlinderung zu erhöhen oder über eine größere Anzahl sympathischer Fasern periphere vasokontriktorische Wirkungen zu intensivieren, so wird für die Fasern, deren Schwellen zuerst überschritten wurden, die Dauer der Überschwelligkeit verlängert und die Dauer der Unterschwelligkeit verkürzt, und bei weiterer Intensitätserhöhung kann sogar die gesamte Modulationsperiode im überschwelligen Bereich liegen.

Mit jeder Intensitätserhöhung werden zwar immer mehr Nervenfasern überschwellig gereizt, aber gleichzeitig erhöht sich für die bereits überschwellig Gereizten die "überschwellige Zeit", so daß schließlich durch Wegfall der Unterbrechung über Erzeugung flüchtiger exzitorischer Aktivität eine Dauerdepolarisation mit entsprechender Blockierung resultieren kann.

Für einen Großteil der Fasern findet somit die Frequenzmodulation nur noch im überschwelligen Bereich statt.

In diesem Falle wird der gewünschte Bereich der reizfrequenzsynchronen Stimulation verlassen und stattdessen der Bereich der flüchtigen exzitatorischen Aktivität ohne eine eindeutige zeitliche Beziehung zur Reizfrequenz und Entladungsfrequenz der gereizten Struktur betreten. Letztlich besteht das Risiko, daß es zu einer Dauerdepolarisation der Zellen, also der Nerven bzw. Muskeln, kommt, die eigentlich gereizt werden sollten. Die lokale Dauerdepolarisation führt bei Nerven zur Leitungsblockierung und bei Muskeln zu einer reversiblen physiologischen Kontraktur.

Aus diesen Gründen können nach der Methode der horizontalen Stimulation maximale tetanische Kontraktionen, wie sie mittels vertikaler Stimulation möglich sind, nicht erzeugt werden.

Ein weiteres Anwendungsgebiet der horizontalen Stimulation besteht gemäß der EP 0 659 099 in der Erzeugung sogenannter Interferenzen. Dabei werden zwei mittelfrequente Ströme, die untereinander eine geringe Frequenzabweichung aufweisen, zur Erzeugung niederfrequenter Interferenzströme überlagert. Im Überlagerungsfeld der erwähnten Mittelfrequenzströme kommt es zu einer Amplitudenmodulation. Die Amplitudenmodulation entsteht durch die Frequenzdifferenz der beiden eingespeisten Mittelfrequenzströme. Das Ziel einer solchen Überlagerung von zwei oder mehr Stromkreisen ist es, die Intensität der Behandlung durch Addition der Einzelintensitäten zu erhöhen, so daß in diesem Bereich Aktionspotentiale oder Wärme erzeugt werden.

Dabei kann durch entsprechende Ansteuerung zwischen Phasen der reinen Wärmebehandlung und der Wärmebehandlung mit Aktionspotentialen und Behandlungspausen unterschieden werden.

In den Behandlungspausen, in denen weder Wärme noch Aktionspotentiale erzeugt werden, verbleibt eine nicht stimulatorische Stoffwechselwirkung, die als sogenannte "grüne Stoffwechselwirkung" bezeichnet wird. Diese Bezeichnung ist als Abgrenzung gegenüber der "gelben Stimulationswirkung" zu verstehen, bei der Nervengruppen gezielt und stimulativ gereizt werden.

Bei der Interferenztherapie werden mittels der betreffenden Elektroden die Stromkreise so angelegt, daß sich das Überlagerungsfeld im jeweiligen Behandlungsgebiet ausbildet.

Ein weiterer Nachteil des aus der EP 0 659 099 vorbekannten Gerätes besteht darin, daß bei der sogenannten langsamen horizontalen Frequenzmodulation relativ lange Pausen der sensiblen Empfindung unvermeidbar sind. In diesen Pausen werden lediglich "grüne Stoffwechselwirkungen" erwartet, die jedoch mit einer höheren Amplitude, als in diesem Verfahren üblich, effizienter genutzt werden könnten.

Zusätzlich zu dem vorstehend beschriebenen Stand der Technik sind inzwischen Geräte auf dem Markt erhältlich, bei denen zwischen Verfahren der vertikalen oder der horizontalen Stimulation gewählt werden kann. Diese jüngere Gerätegeneration ist also zur Durchführung beider Verfahren alternativ in der Lage.

Der Erfindung liegt die Aufgabe zugrunde, ein elektrotherapeutisches Gerät zu schaffen, das die vorstehend erwähnten Nachteile des Standes der Technik vermeidet und einen gleichbleibenden Sicherheitsstandard in Verbindung mit einem erhöhten Behandlungskomfort und einem erweiterten Anwendungsbereich bietet.

Die der Erfindung zugrunde liegende Aufgabe wird durch ein elektrotherapeutisches Gerät gemäß den Merkmalen des Hauptanspruchs gelöst. Vorteilhafte Ausgestaltungen der Erfindung ergeben sich aus den abhängigen Unteransprüchen.

Dadurch, daß gemäß Hauptanspruch ein Behandlungsstrom generiert wird, der innerhalb eines Mittelfrequenzbereiches von 1 - 100 kHz simultan amplituden- und frequenzmoduliert wird, und dieser in den zu behandelnden Körper eingespeist wird, werden einerseits die Vorteile und Möglichkeiten der horizontalen und vertikalen Stimulation auf ein einziges Verfahren vereinigt und überdies die diesen Verfahren jeweils notwendig innewohnenden Nachteile vermieden.

Die mit dem erfindungsgemäßen Gerät durchführbare Elektrotherapie wird in Anlehnung an den ausgewählten Frequenzbereich, der zum größten Teil dem vom gesunden jugendlichen menschlichen Ohr als hohe Töne wahrnehmbaren Frequenzbereich entspricht, als Hochton-Frequenztherapie bezeichnet. Die Auswahl dieses Frequenzbandes stellt sicher, daß die bei niedrigeren Frequenzen verminderte Einkoppelbarkeit von Leistung vermieden wird und der erforderliche Abstand zur Herzkammerflimmern-Schwelle gewahrt bleibt. Höhere Frequenzen würden aufgrund der höheren Schwellen mit den gesetzlich zugelassenen Intensitäten lediglich schwächere oder gar keine neurophysiologischen Reizwirkungen erzeugen und könnten wegen fehlender Stromwahrnehmung das Risiko hitzebedingter Hautschäden erhöhen.

Das erfindungsgemäße Gerät soll in erster Linie zur Behandlung von
- schmerzhaften Gelenkerkrankungen wie Arthrosen
- Rücken- und Nackenschmerzen und entsprechenden Wirbelsäulenerkrankungen,
- Muskelverspannungen,
- Schmerzen und Schwellungen nach Verletzungen und Operationen, unter anderem nach Abklingen der Wirkungen von Lokalanästhetika,
- normalen, verzögerten und ausbleibenden Heilungsprozessen, z.B. Blutergüssen, Knochenbrüchen, Unterschenkel- und Druck-Geschwüren,
- Venenerkrankungen und Ödemen
eingesetzt werden. Außerdem können in Verbindung mit dem erfindungsgemäßen Gerät lokale Betäubungen erzeugt werden.

Das erfindungsgemäße elektrotherapeutische Gerät wirkt sich durch den elektrochemischen Schütteleffekt zusätzlich stoffwechselerleichternd aus, da es durch eine Förderung von Diffusionsvorgängen zu einer Verbesserung der Ver- und Entsorgung von lebendem Gewebe, zu einer Erhöhung der Wahrscheinlichkeit der Begegnung von Enzym und Substrat, zu einer Erhöhung der Wahrscheinlichkeit der Erzeugung von Resonanzphänomenen sowie zur Erzeugung von hormon-imitierenden Wirkungen über die Beeinflussung der cAMP-Bildung in den Zellen kommt.

Durch Nutzung unmodulierter Hochton-frequenzströme kann es zur Erzeugung lokaler Nevenblockaden - z.B. in der Schmerztherapie oder zur Lokalanästhesie - eingesetzt werden. Darüber hinaus können mit dem erfindungsgemäßen Gerät erheblich höhere Leistungseinkopplungen unter Beachtung der lokalen Verträglichkeit erreicht werden.

Dabei erfolgt die simultane Modulation der Amplitude und Frequenz des Behandlungsstromes derart, daß zwischen einem ersten Grenzwert mit niedriger Frequenz f_{U} und einer oberen oder unteren Amplitude A_{O} oder A_{U} des Behandlungsstroms i_{B} und einem zweiten Grenzwert mit hoher Frequenz f_{O} und einer unteren oder oberen Amplitude A_{U} oder A_{O} des Behandlungsstroms i_{B} hin und her verfahren wird, wobei zu der individuellen Reizschwelle des behandelten Patienten entweder im wesentlichen parallel oder diese in einem vorgebbaren Winkel gekreuzt wird.

Innerhalb des Mittelfrequenzbereichs wird mit Vorteil ein bis zu drei Oktaven umfassendes Frequenzband für die Durchführung der Behandlung angegeben, das sich von 4.096 bis 32.768 Hz, also überwiegend im hörbaren Bereich, und somit in einem Bereich zwischen einem hörbaren sehr hohen C und dem ersten mit Sicherheit nicht mehr hörbaren C liegt. Um therapeutisch nutzbare Resonanzphänomene zu erzeugen, sollte das Frequenzband zumindest eine Oktave betragen.

In vorteilhafter Ausgestaltung ermöglicht das elektrotherapeutische Gerät ein Behandlungsverfahren, bei dem die Strombehandlung innerhalb des Frequenzbandes konstant leicht unter- oder überschwellig frequenz- und amplitudenmoduliert durchgeführt wird.

Dabei erfolgt die Einspeisung des Behandlungsstromes über eine Steuerung/Regelung der Spannung bzw. des Stroms derart, daß der Behandlungsstrom der Schwelle leicht unter- oder überschwellig nachgeführt wird.

Hierzu kann das Gerät mit Vorteil derart parametriert werden, daß zunächst ein erster Grenzwert bei einer unteren Frequenz derart eingestellt wird, daß die Stromamplitude bei gleichbleibender Frequenz solange gesteigert wird, bis der Patient eine leichte Empfindung verspürt. Dieser Wert wird als unterer Grenzwert im Arbeitsspeicher des elektrotherapeutischen Gerät abgespeichert. Anschließend wird bei einer vordefinierten oberen Frequenz ein oberer Grenzwert dadurch eingestellt, daß bei konstanter oberer Frequenz die Strom- oder Spannungsamplitude solange erhöht wird, bis erneut eine leichte Stromempfindung durch den Patienten festgestellt wird. Dieser Wert wird als oberer Grenzwert abgespeichert. Die bei einer doppelt-logarithmischen Darstellung des Stroms bzw. der Spannung über der Frequenz sich ergebende leicht ansteigende Kurve ist die Kurve des Behandlungsstromes. Der frequenzabhängige Behandlungsstrom wird nun für die Dauer der vorgebbaren Behandlungszeit frequenz- und amplitudenmoduliert dieser Kurve angepaßt eingespeist.

Die Therapie wird dann durch Abfahren des Frequenzbandes in diskreten Frequenzschritten durchgeführt. Die jeweils vorgegebenen Frequenzschritte werden für einen definierten Zeitraum angelegt. Dabei können die Einwirkungszeiten der einzelnen eingestellten Frequenzschritte durchaus unterschiedlich sein.

In alternativer Ausgestaltung kann wie schon erwähnt wurdemittels des elektrotherapeutischen Gerätes auch im Wechsel eine über- und unterschwellige Durchflutung der zu behandelnden erregbaren Zellen durchgeführt werden. Dabei kann der Kreuzungswinkle der den Behandlungsstom in doppelt logithmischer Darstellung repräsentierenden Gerade mit der Reiuzschwelle mit Vorteil in Abhängigkeit der gewünschten oder erforderlichen Reizung verstellt werden. Ein großer Kreuzungswinkel - also etwa im Bereich zwischen 45 und 90 Grad - steht für eine "harte Reizung"; ein geringer Kreuzungswinkel - also etwa im Bereich zischen 0 und 45 Grad - steht für eine "weiche Reizung". Eine harte Reizung ist beispielsweise zur Schmerzüberlagerung durch Erzeugung einer Gegenirritation gewünscht, eine weiche Reizung, wenn gezielt einzelne Muskelgruppen angesprochen sein sollen

Dabei kann erneut auch hier eine Parametrierung des elektrotherapeutischen Gerätes derart erfolgen, daß im Bereich einer vorgegebenen unteren Grenzfrequenz ein erster Grenzwert mit einer einer überschwelligen Wirkung entsprechenden Spannungsamplitude angefahren und abgespeichert wird und im Folgenden ein zweiter Grenzwert bei einer als oberer Grenzfrequenz bezeichneten Frequenz mit einer einer unterschwelligen Wirkung entsprechenden Spannungsamplitude als oberer Grenzwert abgespeichert wird.

Dabei stellt wie schon erwähnt die Amplitude des Behandlungsspannung über der logarithmisch aufgetragenen Frequenz des Behandlungsstroms eine die Reizschwelle kreuzende Gerade dar.

Auch hier wird das zur Behandlung vorgesehene definierte Frequenzband in definierten Frequenzschritten abgefahren, wobei jeweils ausgewählte Frequenzen durchaus unterschiedlichen Behandlungszeiten zugeordnet sein können.

Das elektrotherapeutische Gerät weist zur Ausführung der vorgenannten Verfahren wenigstens einen Stromgenerator zur Erzeugung des Behandlungsstromes, einen Oszillator und einen Frequenzsteller zur Frequenzmodulation sowie ein entsprechendes Stellglied zur Amplitudenmodulation des Behandlungsstromes auf. Zusätzlich ist eine Prozessoreinheit mit einer entsprechenden Speichereinheit zur selbsttätigen Durchführung der genannten Behandlungsschritte sowie zur Abspeicherung der benötigten Grenzwerte vorgesehen. Die Einspeisung der im Sinne der Erfindung modulierten Behandlungsströme erfolgt über wenigstens zwei mit dem elektrotherapeutischen Gerät verbundene Flächenelektroden.

Das elektrotherapeutische Gerät kann zusätzlich mit einer Zeitsteuerung sowie einer Abschaltautomatik versehen sein.

In vorteilhafter Ausgestaltung ist das elektrotherapeutische Gerät mit mehreren, wenigstens zwei voneinander unabhängig steuer- oder regelbaren Stromkreisen versehen. Hierdurch können einerseits mehrere Patienten gleichzeitig und voneinander unabhängig mit unterschiedlichen Verfahren behandelt werden.

Darüber hinaus können mit derartigen Geräten die bereits erwähnten Interferenz-Verfahren durchgeführt werden.

In besonders vorteilhafter Ausgestaltung ist das elektrotherapeutische Gerät mit vier unabhängigen Stromkreisen zur Erzeugung von vier unterschiedlichen Behandlungsströmen versehen.

Dabei sollte der Frequenzunterschied zwischen den einzelnen Strömen äußerst gering in einem Bereich zwischen 1/60- und 1/5 Hz liegen. Im Unterschied zu herkömmlichen Interferenzstromgeräten zur Erzeugung niederfrequenter Amplitudenmodulationen stellt der geringe Frequenzunterschied eine extrem langsame Periodik bereit. Ursache hierfür ist eine entsprechend langsame Änderung der Verlaufsrichtung der mittels der angelegten Stromkreise in dem zu behandelnden Körper jeweils erzeugten Feldlinien. Die Periodik der Änderung der Feldlinien entspricht etwa derjenigen der Vasomotion. Diese sogenannte langsame Stereo-Interferenz ermöglicht die periodische Einbeziehung unterscheidbarer zu behandelnder Körperbereiche.

Die Erzeugung derart geringer Frequenzdifferenzen der unterschiedlichen einzuspeisenden Ströme ist mit erheblichem regelungstechnischem Aufwand verbunden. Dieser kann dadurch vermieden werden, daß man anstelle der sogenannten "echten Interferenz" mit einer Methode der allmählichen Phasenverschiebung zwischen den drei Strömen arbeitet.

In abermaliger Weiterbildung der Erfindung können die im Wege der sogenannten Hochton-Frequenzbehandlung eingesetzten Frequenzen auch audiophil wiedergegeben werden.

Das elektrotherapeutische Gerät ist hierzu mit einem entsprechenden Gerät zur Wiedergabe der eingesetzten Frequenzen verbunden.

Zusätzlich oder alternativ können sogar ausgewählte Musikstücke zur Erzeugung der Behandlungsströme eingesetzt werden.

Hierzu ist das elektrotherapeutische Gerät mit einem Hochpaßfilter zur Ausfilterung der kritischen niedrigen Frequenzen sowie der Verstärker zur Erzeugung des Behandlungsstromes mit einer entsprechenden Leistungsbegrenzung versehen, um etwaig im Wege der Dynamik von Musikstücken auftretende kritische Leistungsgrößen zu begrenzen.

Die vorstehend beschriebene Musiktherapie, die der vom Körper empfundenen Reizung einen audiophilen Sinneseindruck zur Seite stellt, kann durch den Einsatz von Visualisierungen gesteigert werden.

Hierzu ist das elektrotherapeutische Gerät mit einem Steuergerät zur Ansteuerung einer Beleuchtungsanlage mit wechselnden Farben zur Farbvisualisierung der eingespeisten Frequenzen wirkverbunden.

Die Erfindung wird nachstehend anhand der Zeichnung näher erläutert. Es zeigen:
- Fig. 1: ein Blockschaltbild des elektrotherapeutischen Gerätes,
- Fig. 2: ein Diagramm zur unterschwelligen Behandlung
- Fig. 3: ein Diagramm zur über- und unterschwelligen Behandlung und
- Fig. 4: ein weiteres elektrotherapeutisches Gerät im Blockschaltbild.

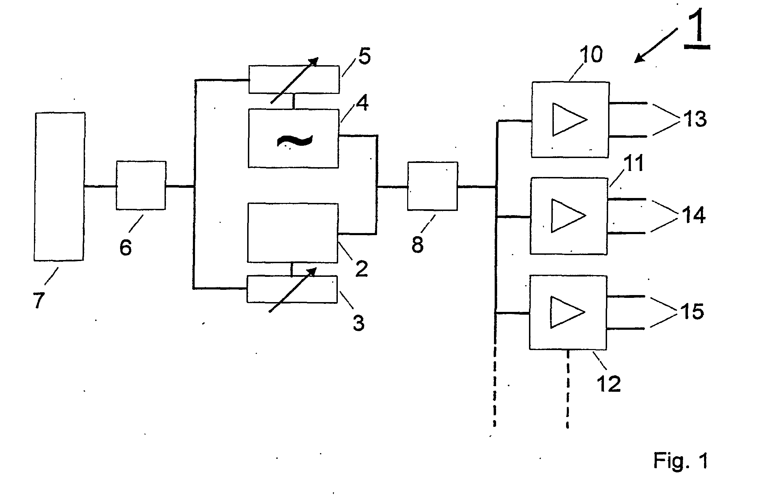

In Fig. 1 ist in Form eines Blockschaltgerätes ein elektrotherapeutisches Gerät 1 gezeigt. Das Gerät 1 umfaßt einen Stromgenerator 2, wobei die Amplitude des Stromes über ein Stellglied 3 einstellbar ist. Darüber hinaus ist ein Oszillator 4, der mit einem Frequenzsteller 5 verbunden ist, vorgesehen. Das Stellglied 3 und der Frequenzsteller 5 werden zumindest indirekt über die Ausgänge eines Mikroprozessors 6, der mit einem Arbeitsspeicher 7 in Datenverbindung steht, angesteuert. Über den Stromgenerator 2 und den Frequenzsteller 5 wird in Abhängigkeit von einem Behandlungsprogramm, das mittels des Mikroprozessors 6 in Verbindung mit einem in Arbeitsspeicher 7 abgelegten Werten bzw. den infolge einer Parametrierung in den Arbeitsspeicher 7 eingespeicherten Werten gesteuert wird, ein Behandlungsstrom I_{B}, dessen Amplitude A bzw. dessen Intensität über das Stellglied 3 und dessen Frequenz f über den Frequenzsteller 5 eingestellt werden, eingespeist. Der derart erzeugte Behandlungsstrom I_{B} kann zur Einspeisung in drei oder mehr unterscheidbare Stromkreise, die mittels einer Superpositionseinrichtung 8 generiert werden, genutzt werden.

Jeder Stromkreis weist hierzu eine eigene Verstärkereinheit 10 bis 12 mit eigener Frequenzerzeugung mittels geeigneter Stellglieder auf. Die Frequenzen können völlig unabhängig voneinander oder in definierter Beziehung zueinander generiert werden.

An die Verstärkerausgänge sind Flächenelektroden 13 bis 15 zur Verbindung mit dem zu behandelnden Körper anschließbar.

Anstelle der in Fig. 1 gezeigten drei Stromkreise kann das elektrotherapeutische Gerät auch mit einem einzigen oder noch mehr unabhängigen Stromkreisen versehen sein.

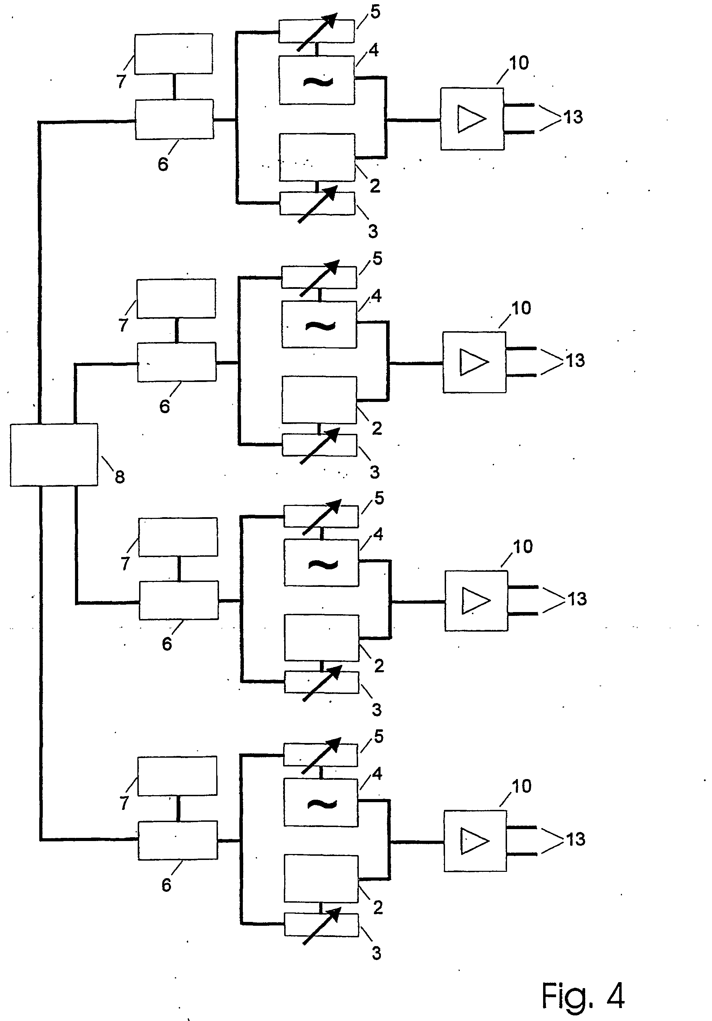

Dabei können die unabhängigen Stromkreise auch gänzlich autark, also nicht mittels einer Superposition ausgebildet sein. Eine gerätetechnische Ausgestaltung dieser Variante ist in Fig. 4 dargestellt.

Nachstehend werden zwei bevorzugte Behandlungsmethoden der elektrotherapeutischen Therapie zur Durchführung mit dem elektrotherapeutischen Gerät 1 beschrieben.

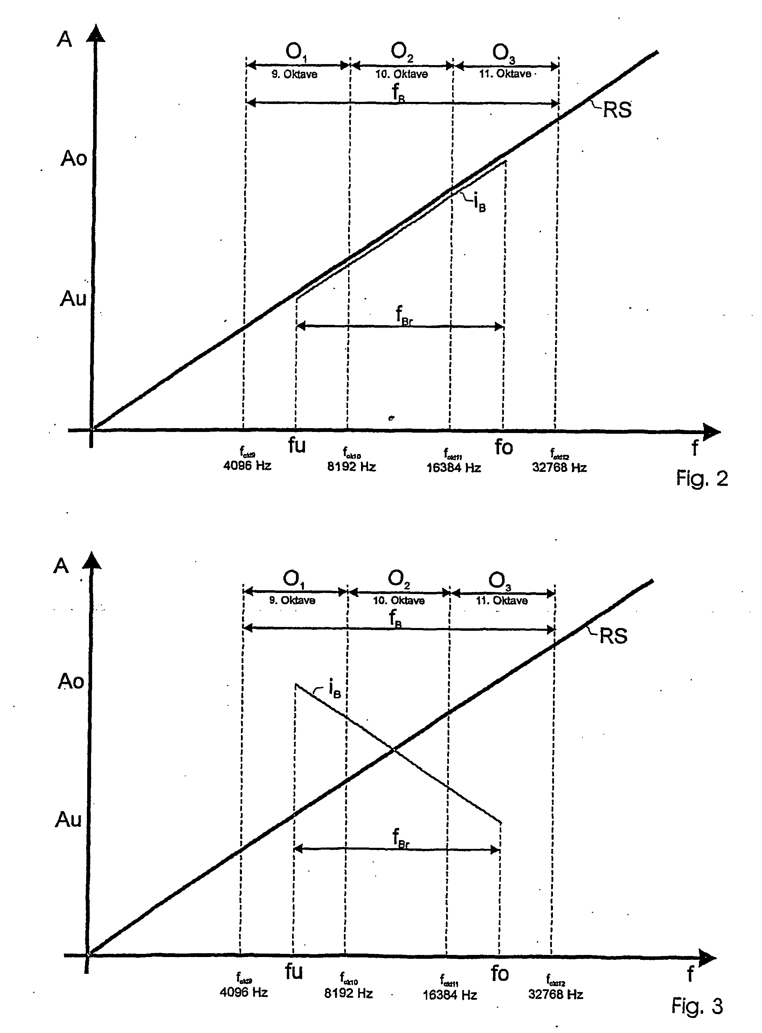

Fig. 2 zeigt ein Verfahren der sensibel unterschwelligen Therapie. Bei dem Diagramm in Fig. 2 ist die Stromamplitude A über der in logarithmischem Maßstab aufgetragenen Frequenz f dargestellt. Die Behandlung findet innerhalb eines Mittelfrequenzbereichs von 4.096 Hz bis 32.768 Hz statt, der als Behandlungsfrequenzband F_{B} bezeichnet ist. Das Behandlungsfrequenzband f_{B} umfaßt drei Oktaven O₁ - O₃, innerhalb derer die Behandlungsfrequenz variiert werden kann. Innerhalb dieses Stromamplitudenfrequenzkoordinatensystems in logarithmischer Darstellung läßt sich die patientenabhängige individuelle Reizschwelle RS als Gerade eintragen.

Die Reizschwelle RS bezeichnet den frequenz- und amplitudenabhängigen Grenzwert der erregbaren Zellen, oberhalb dessen selbsttätig mindestens ein Aktionspotential erzeugt wird. Innerhalb des maximal möglichen Frequenzbandes f_{B} kann je nach Patient und Behandlungstherapie ein oberer und unterer Frequenzwert f_{U} und f_{O} ausgewählt werden, innerhalb derer der Behandlungsstrom I_{B} amplituden- und frequenzmoduliert wird.

Dabei wird zunächst eine Parametrierung derart durchgeführt, daß bei feststehender unterer Grenzfrequenz f_{U} die Spannungs- bzw. Stromamplitude A soweit gesteigert wird, bis eine erste Empfindung des Patienten in dem zu behandelnden Bereich stattfindet. Anschließend wird die Amplitude wieder leicht reduziert, so daß die Empfindung wieder verschwindet; dieser Stromamplitudenwert A_{U} liegt somit kurz unterhalb der Reizschwelle RS. Sobald dieser Wert eingestellt ist, wird dieser als unterer Grenzwert gespeichert. Anschließend wird bei einer feststehenden oberen Frequenz f_{O} erneut die Spannungs- bzw. Stromamplitude A gesteigert, bis der Patient erneut eine leichte Empfindung bemerkt. Danach wird die Amplitude wieder leicht reduziert, so daß die Empfindung wieder verschwindet. Dieser Stromamplitudenwert A_{O} wird als oberer Grenzwert ebenfalls abgespeichert.

Das zwischen der oberen und unteren Grenzfrequenz liegende Frequenzband stellt das simultan für die durchzuführende Behandlung relevante Frequenzband F_{BR} dar. In diesem Frequenzband F_{BR} wird nun in diskreten Frequenzschritten der Behandlungsstrom I_{B} derart moduliert, daß von einem unteren Grenzwert im Bereich der unteren Grenzfrequenz f_{U} mit einer niedrigen Amplitude A_{U} der Strom in den Bereich höherer Frequenzen bis zum Erreichen der oberen Grenzfrequenz f_{O} verschoben wird. Dabei wird mit zunehmender Frequenz auch die Stromamplitude A bis zum Erreichen eines Wertes A_{O} gesteigert. Hierbei wird der Strom parallel zur Empfindungsschwelle innerhalb des relevanten Frequenzbandes f_{BR} erhöht bzw. reduziert. Dabei können die Einwirkungsdauern der einzelnen Frequenzen zugeordneten Spannungs- bzw. Stromamplituden A durchaus unterschiedlich gewählt werden.

Im Wege dieser Therapie wird der Stoffwechsel des Körpers auf verschiedenen Wirkebenen günstig beeinflußt bzw. befördert und bewußt auf eine Reizwirkung verzichtet.

Bei dem im Diagramm nach Fig. 3 dargestellten Verfahren soll neben der Beförderung des Stoffwechsels zusätzlich eine Reizwirkung ausgeübt werden.

Dabei wird Behandlungsstrom I_{B} derart parametriert, daß zunächst im Bereich der unteren Grenzfrequenz f_{U} eine Spannungs- bzw. Stromamplitude A_{O} des Behandlungsstromes I_{B} derart gewählt wird, daß in diesem Bereich eine maximale Amplitude A_{O} deutlich oberhalb der Reizschwelle eingestellt ist. Anschließend wird im Wege der Parametrierung eine obere Grenzfrequenz f_{O} angefahren, wobei sich der der oberen Grenzfrequenz f_{O} zugeordnete Amplitudenwert A_{U} des Behandlungsstromes I_{B} im Verhältnis aus dem der unteren Frequenz zugeordneten Stromamplitude A_{O} ergibt. Beispielsweise kann die untere Amplitude A_{U} 50 Prozent der oberen Amplitude A_{O} des Behandlungsstromes i_{B} betragen. Jedenfalls liegt die der oberen Grenzfrequenz f_{O} zugeordnete untere Amplitude A_{U} deutlich unterhalb der Reizschwelle RS. Der Behandlungsstrom i_{B} wird im Laufe der Behandlung innerhalb des relevanten Frequenzbandes f_{BR} so moduliert, daß sich Phasen der Über- und der Unterschwelligkeit mit der entsprechenden frequenz- und amplitudenmodulationsfrequenzsynchronen Auslösung von Aktionspotentialen periodisch ablösen. Auch hierbei wird das relevante Frequenzband f_{BR} in diskreten Schritten durchlaufen, wobei die Einwirkdauer der den einzelnen Frequenzen zugeordneten Stromamplituden durchaus unterschiedlich gewählt sein kann.

Darüber hinaus können auch die einander abwechselnden Durchläufe durch das relevante Frequenzband f_{BR} unterschiedlich schnell erfolgen. Im vorliegenden Beispiel umfaßt das relevante Frequenzband zwei Oktaven.

In beiden nur beispielhaft ausgewählten Verfahren liegt der jeweils eingestellte Frequenzbereich zumindest überwiegend im hörbaren Bereich. Im Sinne einer ganzheitlichen Behandlung kann es daher sinnvoll sein, das Elektrotherapiegerät 1 zusätzlich mit Geräten zur Wiedergabe der hörbaren Frequenzen zu versehen und diese dem Patienten zugänglich zu machen. Alternativ ist sogar der umgekehrte Weg denkbar, daß ausgewählte Musikstücke zur Ansteuerung des elektrotherapeutischen Gerätes derart eingesetzt werden, daß die Behandlungsströme i_{B} in Abhängigkeit von der harmonischen Gestaltung der ausgewählten Musikstücke ausgewählt werden. In diesem Falle muß dem elektrotherapeutischen Gerät ein Hochpaßfilter zur Aussonderung bzw. intensitätsmäßigen Abschwächung der lokal und systemisch weniger verträglichen niedrigen Frequenzen und den Verstärkereinheiten 10 bis 12 eine Leistungsbegrenzung zugeordnet sein um zu verhindern, daß im Wege der Dynamik von einzelnen Musikstücken die Körperverträglichkeit durch Einkopplung zu großer Leistungen überschritten wird.

In weiterer Ausgestaltung des erwähnten ganzheitlichen Therapiegedankens kann dem elektrotherapeutischen Gerät zusätzlich eine Einrichtung zur Visualisierung der eingesetzten Frequenzen beispielsweise im Wege von entsprechend angesteuerten Farblichtspielen zugeordnet sein.

Somit ist vorstehend ein elektrotherapeutisches Gerät beschrieben, das die Vorteile der horizontalen und vertikalen Stimulation auf sich vereinigt und bei einem gesteigerten Sicherheitsstandard höchsten Bedienkomfort in Verbindung mit neuen Anwendungsmöglichkeiten für den Patienten bietet.

### BEZUGSZEICHENLISTE

- 1: Elektrotherapeutisches Gerät
- 2: Stromgenerator
- 3: Stellglied
- 4: Oszillator
- 5: Frequenzsteller
- 6: Mikroprozessor
- 7: Arbeitsspeicher
- 8: Superpositionseinrichtung
- 10 bis 12: Verstärker
- 13 bis 15: Flächenelektroden

- i_{B}: Behandlungsstrom
- A: Amplitude
- A_{O}: Obere Amplitude
- A_{U}: Untere Amplitude
- f: Frequenz
- f_{B}: Behandlungsfrequenzband
- f_{Br}: relevantes Frequenzband
- O₁ bis O₃: Oktaven
- ES: Empfindungsschwelle
- f_{O}: obere Grenzfrequenz
- f_{U}: untere Grenzfrequenz

## Patentansprüche

1. Elektrotherapeutisches Gerät zur Behandlung des vorzugsweise menschlichen Körpers mit elektrischen Strömen definierter Frequenz und Amplitude mit wenigstens zwei mit dem zu behandelnden Körper zum Schluß eines Stromkreises über diesen Körper verbindbaren Flächenelektroden, bei dem ein Behandlungsstrom (i_{B}), dessen Amplitude (A) und Frequenz (f) simultan modulierbar ist, in den zu behandelnden Körper einspeisbar ist,
**dadurch gekennzeichnet, daß**
der Behandlungsstrom (i_{B}) innerhalb eines als Mittelfrequenzbereich bezeichneten Frequenzbandes (f_{B}) zwischen 1 bis 100 kHz derart modulierbar ist, daß zwischen einem ersten Grenzwert mit einer niedrigen Grenzfrequenz (f_{U}) bei gleichzeitig einer minimalen Amplitude (A_{U}) des Behandlungsstromes (i_{B}) und einem zweiten Grenzwert mit einer oberen Grenzfrequenz (f_{O}) mit einer maximalen Amplitude (A_{O}) des Behandlungsstromes (i_{B}) oder einer maximalen Amplitude (A_{O}) des Behandlungsstromes (i_{B}) mit einer niedrigen Grenzfrequenz (f_{U}) und einem zweiten Grenzwert mit einer oberen Grenzfrequenz (f_{O}) mit einer minimalen Amplitude (Aᵤ) des Behandlungsstromes (i_{B}) in vorzugsweise diskreten Frequenzschritten verfahren wird, wobei mit steigender Frequenz die Amplitude steigt oder sinkt und vice versa mit sinkender Frequenz die Amplitude (A) des Behandlungsstroms (i_{B}) sinkt oder steigt.

2. Elektrotherapeutisches Gerät nach Anspruch 1, **dadurch gekennzeichnet, daß** der Behandlungsstrom (i_{B}) derart moduliert ist, daß zumindest innerhalb eines Teils des Behandlungsfrequenzbandes (f_{B}) zwischen dem ersten und zweiten Grenzwert derart etwas über- oder unterschwellig bezogen auf eine Reizschwelle (RS) - im wesentlichen parallel der Reizschwelle (RS) - verfahren wird, daß der Behandlungsstrom (i_{B}) ständig der von der Amplitude und Frequenz abhängigen Empfindungsschwelle (ES) jeweils etwas über- oder unterschreitend nachgeführt ist.

3. Elektrotherapeutisches Gerät nach Anspruch 2, **dadurch gekennzeichnet, daß** eine Parametrierung des elektrotherapeutischen Geräts (1) derart erfolgt, daß bei einer vorgebbaren unteren Frequenz (f_{U}) die Amplitude (A) des Behandlungsstromes (i_{B}) bis knapp ober- oder unterhalb der Empfindungsschwelle (ES) bis zu einem Amplitudenwert (A_{U}) gesteigert wird und dieser als erster Grenzwert innerhalb eines Arbeitsspeichers (7) des elektrotherapeutischen Geräts speicherbar ist und bei einer vorgebbaren oberen Grenzfrequenz (f_{U}) erneut die Amplitude (A) des Behandlungsstromes (i_{B}) bis knapp ober- oder unterhalb der Emfindungsschwelle (ES) bis zu einem Amplitudenwert (A_{O}) des Behandlungsstromes (i_{B}) gesteigert wird und innerhalb des Arbeitsspeichers (7) als zweiter Grenzwert speicherbar ist.

4. Elektrotherapeutisches Gerät nach Anspruch 2 oder 3, bei dem zwischen dem ersten und zweiten Grenzwert die Nachführung in definierten Frequenzschritten, denen jeweils eine knapp ober- oder unterhalb der Empfindungsschwelle (RS) liegende Stromamplitude (A) zugeordnet ist, erfolgt.

5. Elektrotherapeutisches Gerät nach einem der vorhergehenden Ansprüche, **dadurch gekennzeichnet, daß** der Behandlungsstrom (i_{B}) derart moduliert ist, daß zumindest innerhalb eines Teils des Frequenzbandes (f_{B}) zwischen dem ersten Grenzwert mit einer unteren Frequenz (f_{U}), dem eine oberhalb der Empfindungsschwelle (RS) liegende Amplitude (A_{O}) des Behandlungsstromes zugeordnet ist und einem zweiten Grenzwert mit einer oberen Grenzfrequenz (f_{O}), dem eine deutlich unterhalb der Empfindungsschwelle (RS) liegende Amplitude (A_{O}) des Behandlungsstromes (i_{B}) zugeordnet ist, derart verfahren wird, daß die Reizschwelle (RS) jeweils in einem verstellbar vorgebbaren Winkel gekreuzt wird.

6. Elektrotherapeutisches Gerät nach Anspruch 5, **dadurch gekennzeichnet, daß** eine Parametrierung des elektrotherapeutischen Geräts derart erfolgt, daß bei einer vorgebbaren unteren Frequenz (f_{U}) eine Amplitude (A_{O}) des Behandlungsstromes (i_{B}) deutlich oberhalb der Reizschwelle (RS) angefahren und als erster Grenzwert innerhalb des Arbeitsspeichers (7) speicherbar ist und anschließend einer oberen Grenzfrequenz (f_{O}) eine deutlich unterhalb der Empfindungsschwelle (RS) liegende Amplitude (A_{U}) des Behandlungsstromes (i_{B}) in Form eines definierten Anteils der Amplitude (A_{O}) bestimmt und innerhalb des Arbeitsspeichers (7) als zweiter Grenzwert abgelegt wird.

7. Elektrotherapeutisches Gerät nach Anspruch 5 oder 6, **dadurch gekennzeichnet, daß** zwischen dem ersten und zweiten Grenzwert in vorzugsweise diskreten Frequenzschritten derart verfahren wird, daß sich die Phasen der überschwelligen und unterschwelligen Reizung periodisch abwechseln.

8. Elektrotherapeutisches Gerät nach einem der Ansprüche 5 bis 7, **dadurch gekennzeichnet, daß** ein zwischen der unteren Grenzfrequenz (f_{U}) und der oberen Grenzfrequenz (f_{O}) liegender relevanter Behandlungsfrequenzbereich (f_{BR}) mit vorgebbaren, vorzugsweise unterschiedlichen Geschwindigkeiten durchlaufbar ist.

9. Elektrotherapeutisches Gerät nach einem der vorhergehenden Ansprüche mit einem Strom- oder Spannungsgenerator (2) zur Erzeugung des Behandlungsstromes (i_{B}), der mittels eines Oszillators (4) und einem diesem zugeordneten Frequenzsteller (5) frequenzmodulierbar und mittels eines Stellgliedes (3) amplitudenmodulierbar ist, und einer Prozessoreinheit (6) sowie einer diesem zugeordneten Speichereinheit (7) zur Steuerung und/oder Regelung der Elektrotherapie sowie wenigstens zwei Flächenelektroden (13 bis 15) zum kontaktierenden Anschluß an einen zu behandelnden Körper.

10. Elektrotherapeutisches Gerät nach Anspruch 8 oder 9, **dadurch gekennzeichnet, daß** es mehrere, wenigstens zwei voneinander unabhängige Stromkreise aufweist.

11. Elektrotherapeutisches Gerät nach Anspruch 10 mit wenigstens drei, vorzugsweise vier unabhängigen Stromkreisen zur Erzeugung von Interferenzen innerhalb des zu behandelnden Körpers derart, daß drei Behandlungsströme (i_{B1} - i_{B3}) mit einer vorzugsweise veränderlichen geringen Frequenzdifferenz, vorzugsweise zwischen 1/60- und 1/5 Hz, simultan in den zu behandelnden Körper einspeisbar sind.

12. Elektrotherapeutisches Gerät nach Anspruch 10 oder 11, **dadurch gekennzeichnet, daß** eine allmähliche Phasenverschiebung zwischen den mittels der unterschiedlichen Stromkreise eingespeisten Spannungen bzw. Ströme (i_{B1} - i_{B3}) einstellbar ist.

13. Elektrotherapeutisches Gerät nach einem der Ansprüche 10 bis 12, **dadurch gekennzeichnet, daß** die jeweils zur Behandlung des Körpers eingesetzten Frequenzen (f) simultan audiophon wiedergebbar sind und/oder definierte Musikstücke zur elektrotherapeutischen Behandlung bzw. zur Modulation des Behandlungsstromes (i_{B}) einsetzbar sind.

14. Elektrotherapeutisches Gerät nach einem der vorhergehenden Ansprüche, **dadurch gekennzeichnet, daß** dem elektrotherapeutischen Gerät ein Gerät zur Farb- und/oder Lichtwiedergabe derart zugeordnet ist, daß die zur Modulation des Behandlungsstromes (i_{B}) eingesetzten Frequenzen simultan zur Ansteuerung der Lichtanlage im Sinne einer Licht- und/oder Farbvisualisierung einsetzbar sind.

## Claims

1. Electrotherapeutic apparatus for treatment of preferably the human body with electrical currents of defined frequency and amplitude, with at least two area electrodes connectible with the body, which is to be treated, for closure of a current circuit via this body, in which a treatment current (i_{B}), the amplitude (A) and frequency (f) can be simultaneously modulated, can be fed into the body to be treated, **characterised in that** the treatment current (i_{B}) can be modulated within a frequency band (f_{B}), which is denoted middle frequency range, between 1 and 100 kHz in such a manner that the procedure is in preferably discrete frequency steps between a first limit value with a lower limit frequency (f_{U}) with at the same time a minimum amplitude (A_{U}) of the treatment current (i_{B}) and a second limit value with an upper limit frequency (f_{O}) with a maximum amplitude (A_{O}) of the treatment current (I_{B}) or a maximum amplitude (A_{O}) of the treatment current (i_{B}) with a lower limit frequency (f_{U}) and a second limit value with an upper limit frequency (f_{O}) with a minimum amplitude (A_{U}) of the treatment current (i_{B}), wherein with increasing frequency the amplitude rises or falls and vice versa with decreasing frequency the amplitude (A) of the treatment current (i_{B}) falls or rises.

2. Electrotherapeutic apparatus according to claim 1, **characterised in that** treatment current (i_{B}) is modulated in such a manner that the procedure at least within a part of the treatment frequency band (f_{B}) between the first and the second limit value is slightly above the threshold or below the threshold with respect to a stimulation threshold (RS) - substantially parallel to the stimulation threshold (RS) - such that the treatment current (i_{B}) constantly tracks the sensitivity threshold (ES), which is dependent on amplitude and frequency, respectively to slightly exceed or fall below that threshold.

3. Electrotherapeutic apparatus according to claim 2, **characterised in that** a parameterisation of the electrotherapeutic apparatus (1) is carried out in such a manner that in the case of a predeterminable lower frequency (f_{U}) the amplitude (A) of the treatment current (i_{B}) is increased until just above or below the sensitivity threshold (ES) as far as an amplitude value (A_{U}) of the treatment current (i_{B}) and this can be stored as first limit value within an operating memory (7) of the electrotherapeutic apparatus, and in the case of a predeterminable upper limit frequency (f_{U}) the amplitude (A) of the treatment current (i_{B}) is again increased until just above or below the sensitivity threshold (ES) as far as an amplitude value (A_{O}) of the treatment current (i_{B}) and can be stored within the operating memory (7) as a second limit value.

4. Electrotherapeutic apparatus according to claim 2 or 3, in which the tracking takes place between the first and second limit values in defined frequency steps, with each of which is associated a current amplitude (A) lying just above or below the sensitivity threshold (RS).

5. Electrotherapeutic apparatus according to one of the preceding claims, **characterised in that** the treatment current (i_{B}) is modulated in such a manner that the procedure at least within a part of the frequency band (f_{B}) between the first limit value with a lower frequency (f_{U}), with which is associated an amplitude (A_{O}) of the treatment current lying above the sensitivity threshold (RS), and a second limit value with an upper limit frequency (f_{O}), with which is associated an amplitude (A_{O}) of the treatment current (i_{B}) lying significantly below the sensitivity threshold (RS), is such that the stimulation threshold (RS) is crossed in each instance at an adjustable predeterminable angle.

6. Electrotherapeutic apparatus according to claim 5, **characterised in that** a parameterisation of the electrotherapeutic apparatus is carried out in such a manner that in the case of a predeterminable lower frequency (f_{U}) there is movement towards an amplitude (A_{O}) of the treatment current (I_{B}) significantly above the stimulation threshold (RS) and the amplitude can be stored as a first limit value within the operating memory (7) and that subsequently an upper limit frequency (f_{O}) determines an amplitude (A_{U}) of the treatment current (i_{B}), which lies significantly below the sensitivity threshold (RS), in the form of a defined proportion of the amplitude (A_{O}) and this is stored within the operating memory (7) as a second limit value.

7. Electrotherapeutic apparatus according to claim 5 or 6, **characterised in that** between the first and second limit values the procedure is in preferably discrete frequency steps such that the phases of the stimulation above the threshold and below the threshold periodically alternate.

8. Electrotherapeutic apparatus according to one of claims 5 to 7, **characterised in that** a relevant treatment frequency range (f_{BR}) lying between the lower limit frequency (f_{U}) and the upper limit frequency (f_{O}) can be run through at predeterminable, preferably different speeds.

9. Electrotherapeutic apparatus according to one of the preceding claims with a current or voltage generator (2) for generating the treatment current (i_{B}), which can be modulated in frequency by means of an oscillator (4) and a frequency setter (5) associated therewith and can be modulated in amplitude by means of a setting element (3), and a processor unit (6) as well as a memory unit (7), which is associated therewith, for control and/or regulation of the electrotherapy as well as at least two area electrodes (13 to 15) for contacting connection with a body to be treated.

10. Electrotherapeutic apparatus according to claim 8 or 9, **characterised in that** it comprises several, at least two mutually independent current circuits.

11. Electrotherapeutic apparatus according to claim 10 with at least three, preferably four, independent current circuits for generating interferences within the body to be treated in such a manner that three treatment currents (i_{B1} - i_{B3}) with a preferably variable, small frequency difference, preferably between 1/60 and 1/5 Hz, can simultaneously be fed into the body to be treated.

12. Electrotherapeutic apparatus according to claim 10 or 11, **characterised in that** a gradual phase displacement is settable between the voltages or currents (i_{B1} - i_{B3}) fed by means of the different current circuits.

13. Electrotherapeutic apparatus according to one of claims 10 to 12, **characterised in that** the frequencies (f) respectively used for treatment of the body are simultaneously reproducible by audiophone and/or defined pieces of music are usable for the electrotherapeutic treatment or for modulation of the treatment current (i_{B}).

14. Electrotherapeutic apparatus according to one of the preceding claims, **characterised in that** an apparatus for reproduction of colour and/or light is associated with the electrotherapeutic apparatus in such a manner that the frequencies used for modulation of the treatment current (i_{B}) are simultaneously usable for control of the light installation in the sense of a light and/or colour visualisation.

## Revendications

1. Appareil électrothérapeutique pour le traitement de préférence du corps humain avec des courants électriques d'une fréquence et amplitude définies avec au moins deux électrodes planes pouvant être reliées au corps à traiter pour former un circuit électrique sur ce corps, où un courant de traitement (i_{B}), dont l'amplitude (A) et la fréquence (f) sont modulables simultanément, peut être introduit dans le corps à traiter,
**caractérisé en ce que**
le courant de traitement (i_{B}) peut être modulé à l'intérieur d'une bande de fréquences (f_{B}) désignée comme plage de fréquences moyennes entre 1 à 100 kHz de façon qu'on procède entre une première valeur limite avec une fréquence limite basse (f_{U}) avec en même temps une amplitude minimale (A_{U}) du courant de traitement (i_{B}) et une seconde valeur limite avec une fréquence limite supérieure (f_{O}) avec une amplitude maximale (A_{O}) du courant de traitement (i_{B}) ou une amplitude maximale (A_{O}) du courant de traitement (i_{B}) avec une fréquence limite basse (f_{U}) et une seconde valeur limite avec une fréquence limite supérieure (f_{O}) avec une amplitude minimale (A_{U}) du courant de traitement (i_{B}) de préférence selon des pas de fréquence discrets, où lors d'une fréquence qui monte, l'amplitude monte ou descend et vice versa avec une fréquence qui diminue, l'amplitude (A) du courant de traitement (i_{B}) diminue ou augmente.

2. Appareil électrothérapeutique selon la revendication 1, **caractérisé en ce que** le courant de traitement (i_{B}) est modulé de telle sorte qu'au moins à l'intérieur d'une partie de la bande de fréquence de traitement (f_{B}) entre la première et la seconde valeur limite, on procède de telle sorte légèrement au-dessus ou en dessous du seuil par rapport à un seuil d'excitation (RS) - sensiblement parallèlement au seuil d'excitation (RS) - que le courant de traitement (i_{B}) est guidé constamment pour être légèrement au-dessus ou en dessous du seuil de sensibilité (ES) dépendant de l'amplitude et de la fréquence.

3. Appareil électrothérapeutique selon la revendication 2, **caractérisé en ce qu'**un paramétrage de l'appareil électrothérapeutique (1) a lieu de telle sorte que lors d'une fréquence inférieure prédéfinissable (f_{U}), l'amplitude (A) du courant de traitement (i_{B}) est augmentée jusqu'à légèrement au-dessus ou en dessous du seuil de sensibilité (ES) jusqu'à une valeur d'amplitude (A_{U}) et que celle-ci peut être stockée comme première valeur limite à l'intérieur d'une mémoire de travail (7) de l'appareil électrothérapeutique, et lors d'une fréquence limite supérieure prédéfinissable (f_{U}), de nouveau l'amplitude (A) du courant de traitement (i_{B}) est augmentée jusqu'à légèrement au-dessus ou en dessous du seuil de sensibilité (ES) jusqu'à une valeur d'amplitude (A_{O}) du courant de traitement (i_{B}) et peut être stockée à l'intérieur de la mémoire de travail (7) comme seconde valeur de seuil.

4. Appareil électrothérapeutique selon la revendication 2 ou 3, où a lieu entre la première et la seconde valeur limite la poursuite selon des pas de fréquence définis, auxquels est associée à chaque fois une amplitude de courant (A) situé légèrement au-dessus ou en dessous du seuil de sensibilité (RS).

5. Appareil électrothérapeutique selon l'une des revendications précédentes, **caractérisé en ce que** le courant de traitement (i_{B}) est modulé de telle sorte qu'au moins à l'intérieur d'une partie de la bande de fréquence (f_{B}) entre la première valeur limite d'une fréquence inférieure (f_{U}), à laquelle est associée une amplitude (A_{O}) du courant de traitement située au-dessus du seuil de sensibilité (RS), et une seconde valeur limite d'une fréquence limite supérieure (f_{O}), à laquelle est associée une amplitude (A_{O}) du courant de traitement (i_{B}) située maintenant en dessous du seuil de sensibilité (RS), on procède de telle sorte que le seuil d'excitation (RS) se croise à chaque fois selon un angle prédéfinissable d'une manière ajustable.

6. Appareil électrothérapeutique selon la revendication 5, **caractérisé en ce qu'**un paramètrage de l'appareil électrothérapeutique a lieu de telle sorte que lors d'une fréquence inférieure prédéfinissable (f_{U}), une amplitude (A_{O}) du courant de traitement (i_{B}) est abordée nettement au-dessus du seuil d'excitation (RS) et peut être stockée comme première valeur limite à l'intérieur de la mémoire de travail (7), et à la suite d'une fréquence limite supérieure (f_{O}), une amplitude (A_{U}) du courant de traitement (i_{B}) située nettement en dessous du seuil de sensibilité (RS) est déterminée sous la forme d'une part définie de l'amplitude (A_{O}) et est stockée à l'intérieur de la mémoire de travail (7) comme seconde valeur de seuil.

7. Appareil électrothérapeutique selon la revendication 5 ou 6, **caractérisé en ce qu'**on procède entre les première et seconde valeurs limites selon des pas de fréquence de préférence discrets de telle sorte que les phases de l'excitation au-dessus du seuil et en dessous du seuil alternent périodiquement.

8. Appareil électrothérapeutique selon l'une des revendications 5 à 7, **caractérisé en ce qu'**une zone de fréquences de traitement importante située entre la fréquence limite inférieure (f_{U}) et la fréquence limite supérieure (f_{O}) peut être traversée à des vitesses prédéfinies, de préférence différentes.

9. Appareil électrothérapeutique selon l'une des revendications précédentes, avec un générateur de courant ou de tension (2) pour produire le courant de traitement (i_{B}), dont la fréquence peut être modulée au moyen d'un oscillateur (4) et d'un régulateur de fréquence (5) associé à celui-ci et dont l'amplitude peut être modulée au moyen d'un organe de positionnement (3), et une unité de processeur (6) ainsi qu'une unité de stockage (7) associée à celle-ci pour la commande et/ou le réglage de l'électrothérapie, et au moins deux électrodes planes (13 à 15) pour la connexion de contact à un corps à traiter.

10. Appareil électrothérapeutique selon la revendication 8 ou 9, **caractérisé en ce qu'**il présente plusieurs, au moins deux circuits électriques indépendants l'un de l'autre.

11. Appareil électrothérapeutique selon la revendication 10, avec au moins trois, de préférence quatre circuits électriques indépendants pour produire des interférences à l'intérieur du corps à traiter, de telle sorte que trois courants de traitement (i_{B1} - i_{B3}) avec une petite différence de fréquence, de préférence modifiable, de préférence entre 1/60 et 1/5 Hz, peuvent être introduits simultanément dans le corps à traiter.

12. Appareil électrothérapeutique selon la revendication 10 ou 11, **caractérisé en ce qu'**un décalage de phase progressif est réglable entre les tensions respectivement courants (i_{B1} - i_{B3}) introduits au moyen des circuits électriques différents.

13. Appareil électrothérapeutique selon l'une des revendications 10 à 12, **caractérisé en ce que** les fréquences (f) utilisables à chaque fois pour le traitement du corps peuvent être reproduites simultanément de manière audiophone et/ou que des pièces de musique définies peuvent être utilisées pour le traitement électrothérapeutique respectivement pour la modulation du courant de traitement (i_{B}).

14. Appareil électrothérapeutique selon l'une des revendications précédentes, **caractérisé en ce qu'**il est associé à l'appareil électrothérapeutique un appareil pour la reproduction de couleur et/ou de lumière de telle sorte que les fréquences utilisées pour la modulation du courant de traitement (i_{B}) peuvent être utilisées simultanément pour commander l'installation lumineuse dans le sens d'une visualisation de lumière et/ou de couleur.
